Europäisches Patentamt

European Patent Office

Office européen des brevets

(11) Publication number: **0 226 593 B1**

(12) **EUROPEAN PATENT SPECIFICATION**

(45) Date of publication of patent specification: **15.01.92**　(51) Int. Cl.⁵: **G01N 27/28, C09K 3/00**

(21) Application number: **86902144.4**

(22) Date of filing: **11.03.86**

(86) International application number:
**PCT/US86/00487**

(87) International publication number:
**WO 86/05590 (25.09.86 86/21)**

(54) **APPARATUS FOR CHEMICAL MEASUREMENT OF BLOOD CHARACTERISTICS.**

(30) Priority: **19.03.85 US 713435**
　　　　**29.08.85 US 770740**

(43) Date of publication of application:
**01.07.87 Bulletin  87/27**

(45) Publication of the grant of the patent:
**15.01.92 Bulletin  92/03**

(84) Designated Contracting States:
**AT BE CH DE FR GB IT LI LU NL SE**

(56) References cited:
**EP-A- 0 189 316**　　**US-A- 3 556 950**
**US-A- 3 681 255**　　**US-A- 3 763 422**
**US-A- 3 874 850**　　**US-A- 4 109 505**
**US-A- 4 116 336**　　**US-A- 4 218 197**
**US-A- 4 225 410**　　**US-A- 4 375 743**
**US-A- 4 415 534**　　**US-A- 4 424 276**
**US-A- 4 452 682**

(73) Proprietor: **MALLINCKRODT SENSOR SYS-
TEMS, INC.**
**1230 Eisenhower Place**
**Ann Arbor, MI 48105(US)**

(72) Inventor: **ENZER, Steven, E.**
**12740 Monroe Pike**
**Brooklyn, MI 49230(US)**
Inventor: **BURGESS, Bruce, M.**
**115 Longman Lane,**
**Ann Arbor, MI 48103(US)**
Inventor: **WYMAN, Jack, S.**
**3267 Springbrook**
**Ann Arbor, MI 48104(US)**
Inventor: **HENDERSHOT, Ricky, G.**
**2319 Sandalwood Circle**
**Ann Arbor, MI 48105(US)**

(74) Representative: **Szczuka, Jan Tymoteusz et al
Cruikshank & Fairweather 19 Royal Ex-
change Square
Glasgow G1 3AE Scotland(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

N, Medical Research Engineering, Issued Second Quarter 1966, Fumio Gotoh et al., Continuous Recording of Human Cerebral Blood Flow and Metabolism.

## Description

### Field of the Invention

This invention relates to apparatus for measuring certain characteristics of a blood sample and more particularly to such apparatus which employs a disposable cartridge containing a bank of sensors for such characteristics and reagents used to calibrate the sensors.

### Background of the Invention

In a variety of clinical situations it is important to measure certain chemical characteristics of the patient's blood such as pH, concentrations of calcium, potassium ions and hematocrit, the partial pressure of $O_2$ and $CO_2$ and the like. (See, for example, Fundamentals of Clinical Chemistry, Tietz, Editor, page 135 et seq., Electrochemistry; page 849 et seq., Blood Gases and Electrolytes; 1976, Saunders Company, Phila.) These situations range from a routine visit of a patient in the physician's office to monitoring during open-heart surgery for which situations the required speed, accuracy and similar performance characteristics vary with each situation.

Measurement of chemical characteristics of blood during open-heart surgery provides the most demanding set of criteria. Presently, blood gas analysis during major surgery is provided by repeated transfer of discrete blood samples to a permanent lab-based blood gas analyzer or by use of sensors placed in-line with the extra-corporeal blood circuit of a heart-lung machine employed to bypass the patient's heart.

The transfer of discrete blood samples, required by blood-gas analyzers inherently increases the risk of contaminating the blood sample with ambient air, which may alter certain of the monitored characteristics. Additionally, since such analyzers are complex and costly devices, they are typically located only in the hospital lab where they need to be operated by a skilled technician, resulting in undesirable delay during surgery, critical care or intensive care. Further, such analyzers employ bubble tonometers to generate a suitable electrolyte referent mixture by dissolving quantities of gases, stored in pressurized free-standing tanks, into the electrolyte solution. While replacement of these gas tanks is infrequently required, it is a cumbersome procedure. Finally, these existing analyzers require cleaning to decontaminate all exposed portions from the prior patient's blood prior to subsequent use.

Although use of in-line sensors minimizes the risk of contamination during transfer and of delay, they have a response which normally varies or "drifts" during use; moreover, this draft is not at a constant rate. Present in-line sensors can only be calibrated before they are placed in the extra-corporeal circuit. Thus, the inherent drift of these in-line sensors cannot be monitored, resulting in readings of ever decreasing reliability as time passes.

European patent specification no. 0189316 discloses apparatus for measuring or detecting a chemical entity in a liquid sample the apparatus comprising a module, reagent chambers, a sample inlet port, a bank of electrodes, means to control a selector valve and a rotary valve and a computer arranged to receive and analyze signals from the electrodes.

### Summary of the Invention

The present invention is directed to a system which provides quick, on-site contemporaneous blood chemistry analysis, with minimal risk of contamination, and which maintains its accuracy over its useful life.

The present invention provides a system for blood chemistry analysis as disclosed in claim 1.

In a preferred embodiment of the present invention there is adapted to be connected to a blood collection device, an extracorporeal shunt, or an ex vivo blood source such as a heart/lung machine used to sustain a patient during surgery, intensive care, critical care and the like. The machine is designed to allow small test samples of flowing live ex vivo blood to be diverted off-line from either the venous or arterial flow lines of a flowing blood source such as a heart/lung machine directly in real time to a chamber exposed to a bank of solid state micro-electrodes which generate electrical signals proportional to chemical characteristics of the real time flowing blood sample.

The bank of electrodes is housed in a disposable cartridge, adjacent to a thermal plate which maintains the test sample at a constant temperature. Upon insertion of the cartridge into a chamber of a suitably adapted blood chemistry analysis machine, the electrodes connect to an electrode interface which selects one of the plurality of electrical signals generated by the sensors and passes the selected signal to a microprocessor in the machine where it is converted from analog to digital form suitable for analysis, storage and display.

A metal plate in the cartridge connects to a thermal unit in the machine which monitors the temperature of and generates and transmits heat to the plate and through it to the sample in the adjacent electrode chamber in order to maintain the sample at a constant temperature.

The cartridge also contains at least one, and preferably two containers of reference or calibrating electrolyte solution (i.e., solution serving for purposes of quality control including calibration, sometimes referred to hereinafter as control or calibration solution), as well as a reservoir suitable to collect waste fluids following assay. Upon insertion of the cartridge, a selection valve in the cartridge connects to a shaft in the machine, controlled by the microprocessor, to selectively allow either of the calibrating solutions or the test sample to flow across the electrodes.

The force driving the fluid flow through the cartridge (eg, by positive pressure or suction) is provided by a peristaltic pump formed when a set of rotatable drive rollers in the machine pinch exposed portions of tubing against the curved wall of the pump slot on the cartridge. The rotation of the rollers forces either the calibrating solutions or a test sample from their respective sources through the cartridge tubing across the electrode chamber and into the waste collection reservoir. The rotation of the drive rollers is controlled by the microprocessor.

In addition to the features already mentioned, the analysis machine houses an internal digital clock which provides a time base for the operation of the system, a back-up battery power source, an operator keyboard, a display and a printer.

In operation, after all connections are suitably made, the selection valve and drive rollers cooperate to cause the calibrating solution to flow into the electrode chamber where a reading is taken and stored in the microprocessor. Subsequently and in a similar manner, a reading of the test sample is taken, analyzed by the microprocessor and displayed. The assayed fluids are directed into the waste collection reservoir. The microprocessor controls and repeats this cycle of calibration and test sample assay at a rate preselected and entered by the operator through the control keyboard. The keyboard also allows the operator to take an immediate assay at any time, even while the machine is in its automatic cycle mode, limited only by the recycling time of between two and three minutes. Following surgery, the cartridge and the tubing connecting the venous and arterial flows of the heart-lung machine to the cartridge are discarded and the machine is ready for use with a new cartridge.

Brief Description of the Drawings

These and other objects and advantages of the present invention will be more apparent upon reading the following specification and by reference to the drawings in which:

FIGURE 1 is a schematic diagram showing the major components of a preferred embodiment of a blood gas analysis system;

FIGURE 2 is an elevated side view of one embodiment of the cartridge useful with the system of FIGURE 1; showing the insertion end of the cartridge in the foreground;

FIGURE 3 is a fragmentary perspective view of this embodiment of the cartridge;

FIGURE 4 is a side view of the trailing end of this embodiment of the cartridge;

FIGURE 5 is an exploded view of the selection valve contained in this embodiment of the cartridge;

FIGURE 6a is a frontal view of the electrode card contained in this embodiment of the cartridge;

FIGURE 6b is a cross sectional view of this electrode card; and

FIGURE 7 is a fragmentary side view of the end wall at the insertion end of this embodiment of the cartridge, showing the peristaltic pump slot;

FIGURE 8 is an elevated side view of that portion of the blood gas analysis machine useful with the system of FIGURE 1, adapted to receive and connect suitably to certain features of this embodiment of the cartridge; and

FIGURE 9 is a frontal view of one embodiment of the control panel of the blood gas analysis machine showing the display and keyboard.

Description of the Preferred Embodiment

While the apparatus for chemical measurement of blood characteristics of the present invention may be used in a variety of clinical and experimental environments, the preferred embodiment of the invention is described as being used in major surgery. This description should not be taken to limit the applicability of the present invention.

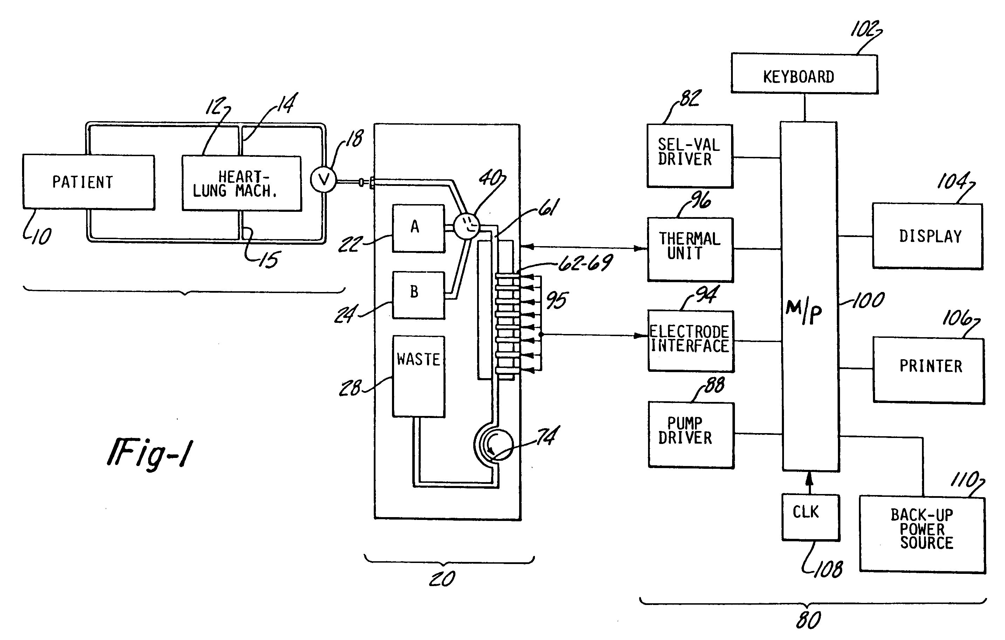

FIGURE 1 shows in schematic form a blood gas analysis system suitable for use during surgery in which a patient 10 is sustained by a heart/lung machine 12. This system allows a test sample of blood to be diverted from either the venous flow 14 or the arterial flow 15 of the heart/lung machine 12, as selected by the system using a two-way valve 18, directly to a cartridge 20 containing a bank of sensing electrodes 62-69. These electrodes 62-69 generate electrical signals proportional to distinct characteristics of the blood sample. These signals are transmitted to a microprocessor 100, contained within a blood chemistry analysis machine 80 into which the cartridge 10 has been inserted. After analyzing these signals, the microprocessor 100 controls a display 104 to display the values of these parameters of the blood sample to provide the surgeon with information on the status of the patient 10.

The system operator uses a keyboard 102 to program the microprocessor 100 with the desired frequency of assays to be made by the system during surgery. The microprocessor 100 then controls the selection valve driver means 82 in the machine 80 to cooperate with a selection valve 40 in the cartridge 20 to allow the sequential fluid flow from a calibrating solution bag 22 and a calibrating solution bag 24, both contained in the cartridge 20, and then from the venous flow 14 or arterial flow 15, into an electrode channel 61 exposed to a bank of electrodes 62-69. The distinct reference solutions from the bags 22 and 24 provide a two-point calibration of the electrodes 62-69. In a similar manner, at the selected intervals, subsequent assays of blood samples are made, most preceded by one-point calibration, with occasional two-point calibration made to ensure continued accuracy. Upon completion of the surgery or depletion of the calibrating solutions, the cartridge 20 is discarded and replaced by a new cartridge 20 for subsequent use of the system.

All of these features and additional features are explained more fully herein.

The Cartridge And Reference Solutions; Blood Facsimile Reference Solution

Referring now to the FIGURES 2 and 3, there is shown a box-like cartridge 20 which is preferably made of rigid plastic. The dimensions of the cartridge allow insertion into a blood gas electrolyte analysis machine 80, shown in FIGURE 1, appropriately engaging features to be described herein.

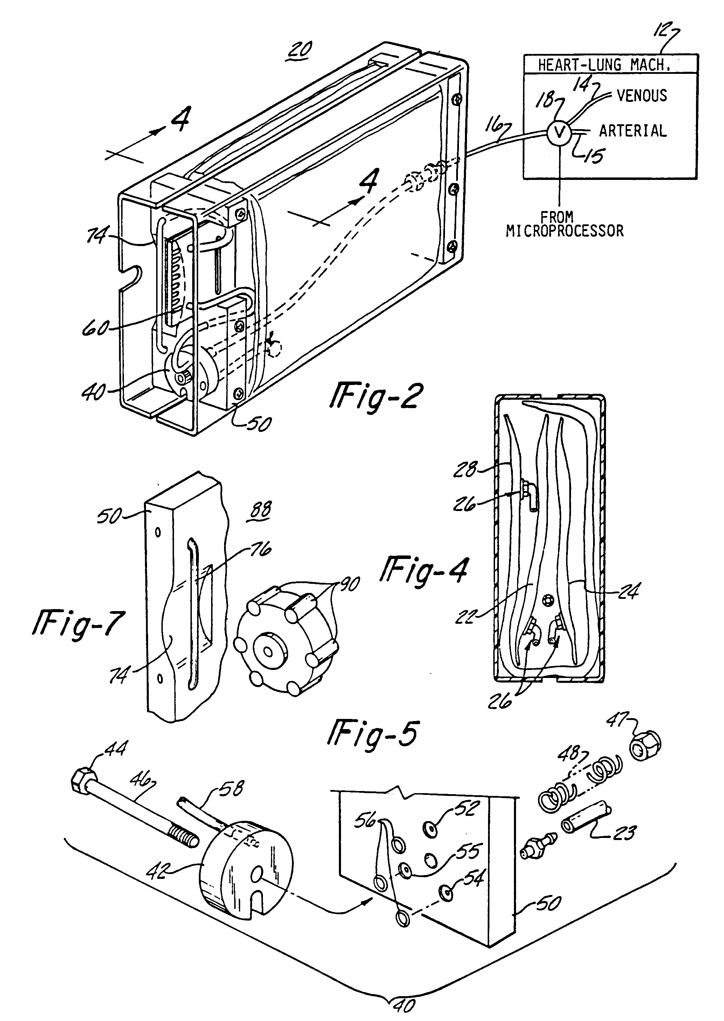

The main body of cartridge 20 is partially enclosed to provide protection of its contents, flexible bags which are two calibrations solution bags 22 and 24, and a waste collection bag 28. The calibrating solution bags 22 and 24 have zero head space and contain solutions and dissolved gases therein that preferably are specially formulated as described hereinafter, having known, distinct electrochemical characteristics. For a description of the technology of packaged reference or calibration solution, see U.S. Patent No. 4,116,336. The third bag 28 or waste collection bag begins in an empty state and is intended to collect waste calibrating solution and blood products following assays. Preferably, the calibrating solution bags 22 and 24 are encircled by the two sides of waste collection bag 28, as shown in FIGURE 4.

The Reference Solutions

The bags 22 and 24 each are gas impermeable and contain an aqueous reference (i.e., calibration or control) solution (a solution electrochemically resembling blood with respect to dissolved gas and electrolyte) having known values of the chemical characteristics over a range of values that the system is intended to monitor. The values of those characteristics are different in the two bags so that by sequential passage of the two calibrating solutions over the electrodes 62-69, a two point calibration or bracket (e.g. high and low) calibration of the measurement characteristics of the electrodes may be made.

In order to maintain the concentration of gases, such as oxygen and carbon dioxide, at a known constant level in the bags 22 and 24, independent of variations in ambient pressure and temperature, the gases are added to the solutions, during their packaging, in a special manner. As a feature of the invention, the packaging in one embodiment to be described is performed under conditions of pressure and temperature which are different from those that will be encountered during normal use of the solutions, so that advantageously the solutions may be fully saturated with the gases at the time of packaging with the important but hitherto unrealized result that these same solutions will be suitably unsaturated during use. Typically, both the temperature will be higher and the ambient pressure lower during the packaging procedure than will ever be encountered in use. For example, for a blood facsimile formulation tonometered with oxygen, $CO_2$ and nitrogen, packaging may occur at a pressure above about 625 mgs. to about 700 mm. Hg and at elevated temperatures in the approximate range from $45^\circ$ to $50^\circ$ C., higher temperatures being unnecessary. During packaging, the liquids are fully saturated with the gases and the packages are sealed in an effort to minimize entrapped air. It is found that later when the temperature and ambient pressure are at normal use values, the packaged solutions will not be saturated but their analyte concentrations will still be at the known values achieved during initial filling process. Since the solutions are unsaturated, there is no tendency for the gases in the solution to outgas into any gas bubbles formed during use.

By way of illustration but without limitation, a preferred embodiment of reference solutions for dual monitoring as described above, comprises the following solutions designated A and B and their respective methods of tonometry.

Formulations And Tonometry Procedure

Calibration Reference Solution A: $Na^+$, $Ca^{++}$, $pCO_2$, pH

Prepared at 37°C and at atmospheric pressure tonometered with 8% $CO_2$ -$N_2$ gas.

All compounds are weighed, combined, and diluted to volume except the calcium salt which is added after tonometering has started.

| COMPOUND | CONCENTRATION | MASS. 1.0 L |
|---|---|---|
| Buffer, 3-Morpholinopropane-sulfonic Acid (MOPS) | 14.0 mmol/l | 2.926 g |
| Buffer, NaMOPS | 36.0 mmol/l | 8.316 g |
| Buffer, $NaHCO_3$ | 14.5 mmol/l | 1.218 g |
| NaCL | 110 mmol/l | 6.430 g |
| $NaN_3$ | .01% w/w | .007 g |
| KCl | 6.0 mmol/l | .447 g |
| $CaCL_2 \cdot 2H_2O$ | 1.25 mmol/l | .184 g |
| 1.0N HCl | ca 8 mmol/l | ca 8 ml |
| 25 wt. % Surfactant (BRIJ 35) aq. soln. | | |

This gives parameter levels of:

| | | | mmol/l | | |
|---|---|---|---|---|---|
| pH | $PCO_2$ mm Hg | $PO_2$ mm Hg | $K^+$-Radiometer | $K^+$-Beckman | Ca++ |
| 7.330-7.345 | 15.5-19.0 | 116-120 | 5.6-5.8 | 5.60-5.75 | .85-.95 |

Calibration Reference Solution B: $Na^+$, $Ca^{++}$, $PO_2$, $PCO_2$, pH

Prepared at 50°C and at 700 mm Hg absolute pressure tonometered with 21% $O_2$ - 4% $CO_2$-$N_2$ gas.

All compounds except the calcium salt are weighed, transferred and combined, and diluted to volume with $H_2O$. The calcium salt is added after tonometering has started.

| COMPOUND | CONCENTRATION | MASS. 1.0 L |
|---|---|---|
| Buffer: Imidazole | 50 mmol/l | 3.405 g |
| $Na_2SO_3$ | 10 mmol/l | 1.260 g |
| $NaHCO_3$ | 11.5 mmol/l | 0.966 g |
| NaCl | 93 mmol/l | 5.44 g |
| $NaN_3$ | .01% w/w | .007 g |
| KCl | 2.0 mmol/l | .149 g |
| $CaCl_2 \cdot 2H_2O$ | 0.25 mmol/l | .037 g |
| 1.0N HCl | 23 mmol/l | 23 ml |
| 25 wt. % Surfactant (BRIJ 35) aq. soln. | 0.25 ml/l | |

This gives parameter levels of:

|  | | | | mmol/l | |
|---|---|---|---|---|---|
| pH | PCO2 mm Hg | PO2 mm Hg | $K^+$-Radiometer | $K^+$-Beckman | CA++ |
| 6.890-6.910 | 44-48 | 0.0 | 1.8-1.9 | 1.83-1.98 | .18-.22 |

Thus, the reference solution in packaged form for use in blood/gas measuring or monitoring equipment according to a preferred embodiment of the invention comprises a flexible gas-impermeable void-free package of an aqueous solution electrochemically resembling arterial blood or venous blood. The solution contains electrolyte (ionic potassium and calcium) and dissolved gas at known partial pressure. The mentioned packaged solution may thus be regarded as an electrochemical facsimile of blood in a stable form such as that exemplified by reference solution B above. The total gas pressure in the packaged solution is in the range from about 0.82 to about 0.92 atmospheres (625 to 700 mm Hg) at use temperature, i.e., temperature encountered during storage and monitoring. The package may be a flexible bag, as indicated, or other suitable container package.

It is found that the preparation of the above-mentioned packaged blood facsimile involves previously unrealized compatibility problems. In this regard, when constituting the solution by conventional tonometry procedures, one finds that the compounds are incompatible in that ionic calcium separates unmanageably from the solution as a non-ionic precipitate.

Therefore, another preferred aspect of the invention resides in a method of producing a packaged blood facsimile reference solution containing oxygen gas, carbon dioxide gas and ionic potassium and calcium, without the unwanted precipitation of calcium. The method comprises constituting an aqueous buffered solution containing ionic potassium at a predetermined concentration, subjecting the resulting solution to tonometry with the gases, and following initiation of tonometry admitting ionic calcium in predetermined amount with the tonometered solution, whereby the resulting solution is stable with respect to the desired ionic parameters and the solution can be suitably packaged.

Still another preferred method aspect of the invention concerns a method of producing a package of an electrochemically stable tonometered blood facsimile solution, as indicated, for storage and for use in blood/gas monitoring at normal atmospheric pressure. The method comprises packaging the solution in a sealed flexible gas-impermeable envelope free of voids (i.e., zero head space) while maintaining the solution at sub-atmospheric pressure and at temperature higher than said use temperature, as described hereinbefore. The packaging can be done in any suitable way by packaging art means which per se may be conventional.

A preferred embodiment of the package aspect of the invention concerns a flexible gas-impermeable package. The package contains a blood facsimile reference solution for use in blood gas electrolyte analysis, comprising ionic potassium and calcium and tonometered with oxygen and carbon dioxide, the package contents being entirely liquid and free of voids or bubbles under conditions of use.

Both calibrating solution bags 22 and 24 contain tube fittings 26, as shown in FIGURE 3 and 4, which connect in turn to their calibrating solution tubes 23 and 25 respectively. The calibrating solution tubes 23 and 25 subsequently connect to a selection valve 40 as described later.

The waste collection bag 28, suitable for collection of waste blood products and calibrating solutions following assay, is formed of a material which is semi-permeable to gases but impermeable to the liquid component of blood and to the calibrating solutions. It is thus intended that only the liquid component of

7

blood and of the calibrating solution will occupy space in the waste collection bag 28. In the preferred embodiment, the bags 22, 24 and 28 are contained in the main body of the cartridge 20 such that as the waste collection bag 28 fills, it will occupy the space created by the emptying of the calibrating solution bags 22 and 24.

The waste collection bag 28 also has a tube fitting 26, shown in FIGURE 4, connected to a waste tube 76, which originates at the discharging end of the electrode card 60. A check valve 77 (FIGURE 3) is disposed in the flow line to the collection bag 28 to prevent back-flow.

The trailing end of the cartridge 20, being the end opposite to the cartridge which is inserted into the blood gas analysis machine 80, contains a blood intake port 30, shown in FIGURES 2 and 3, connected by tubing 16 to either the venous blood flow 14 or the arterial blood flow 15 of a heart/lung machine 12 used to sustain the patient 10 during surgery. The system operator controls the selection of a blood sample from either the venous flow 14 or the arterial flow 15 by use of a valve 18 in the tubing 16. The blood intake port 30 is connected by a blood intake tube 32, passing through the interior of the cartridge 20 between the bags 22 and 24, to the selection valve 40 at the insertion end of the cartridge 20.

As shown in FIGURES 2 and 3, the insertion end of the cartridge 20 includes a selection valve 40, an electrode card 60, a peristaltic pump slot 74, and a metal plate 70. In the preferred embodiment, this insertion end of the cartridge 20 is protected by the overhanging sides and top of the plastic encasing material of the cartridge 20 but is exposed to the connecting portions of the blood gas analysis machine 80.

Referring to FIGURES 2 and 3, the selection valve 40, the electrode card 60, and the peristaltic pump slot 74 are all intended to connect with appropriate contacts in the blood gas analysis machine 80. The insertion end wall 50, formed of plastic, serves to provide partial protection to the bags inside the main body of the cartridge 20, and to provide well-positioned contact between the appropriate portions of the cartridge 20 and the blood gas analysis machine 80 upon insertion of the former.

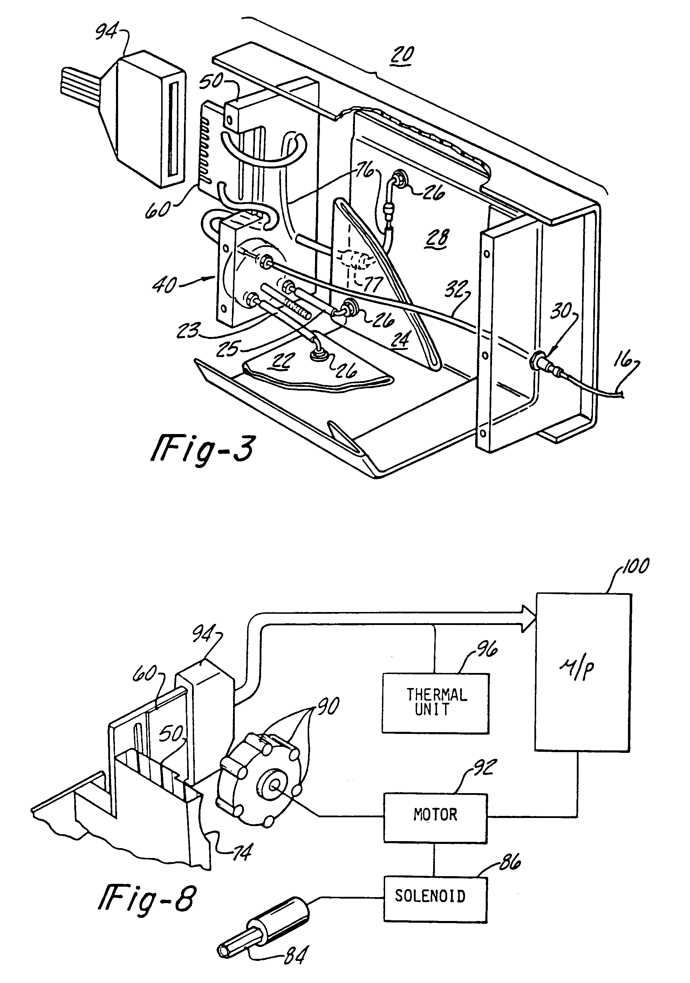

As shown in FIGURE 5, the selection valve 40 has a rotating plug 42, formed of a thick ring of plastic, which houses the electrode input tube 58, running ultimately to the input end of the electrode channel 61. The rotating plug 42 is held flush against the insertion end wall 50 by a bolt 46 passing through the center of the plug 42 and through the end wall 50 into the interior of the cartridge 20. As the bolt 46 extends into the interior of cartridge 20, it passes through a spring 48 which seats against a nut 47 which in turn serves to seat the plug 42 flush against the head 44 of the bolt 46. The spring 48 thus serves to urge the rotating plug 42 against the insertion end wall 50. The exterior end of the bolt head is recess (e.g., Allen-recess) adapted to receive a drive shaft 84 in matching relation when the cartridge 20 is inserted into the machine 80.

The rotating plug 42 seats against that portion of the insertion end wall 58 having three ports 52, 54 and 55. A blood sample port 52 connects in the interior of the cartridge 20 to the blood intake tube 32; the calibration solution ports 54 and 55 connect in the interior of the cartridge 20 to the calibration solution tubes 23 and 25 respectively. Each end of the ports 52, 54 and 55 which contacts the rotating plug 42 is sealed by a rubber ring 56 to provide a leakproof connection to an electrode input tube 58.

As seen in FIGURE 5, the selection valve 40 allows the microprocessor to direct the rotating plug 42 into a position aligning the electrode input tube 58 with either the blood intake tube 32, the calibrating solution tube 23, or the calibrating tube 25; when aligned with one of these tubes, the rotating plug 42 blocks the flow from the other two tubes.

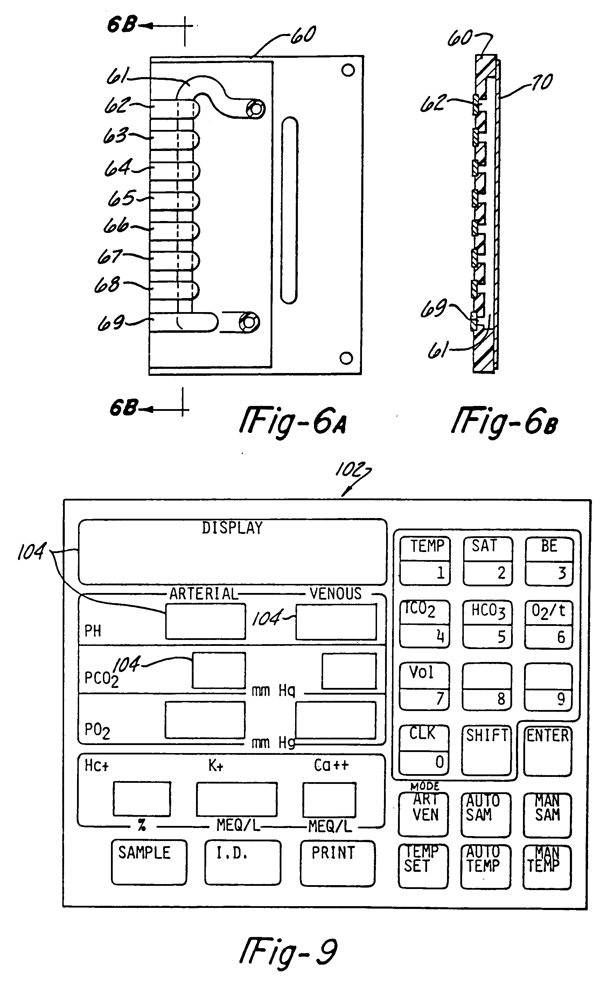

Another feature of the insertion end of the cartridge 20 is the electrode card 60, best shown in FIGURES 6a and 6b. The electrode card 60 is formed of polyvinylchloride in a generally rectangular shape and contains a bank of electrodes 62-69. The electrode card 60 is fastened to the insertion end wall 50 such that the electrode bank protrudes and connects with an electrode interface 94, within the blood gas analysis machine 80.

Preferably, each of the electrodes 62-69 are distinctly formed planar solid state electrodes which allow assay of different characteristics of human blood. The distinct construction of each electrode 62-69 produces a plurality of voltages or currents proportional to different chemical characteristics of a test sample. The electrodes 62-69 are formed in preformed circular slots in the electrode card 60. These solid state electrodes may be either of the ion-selective membrane type, as is preferable, of the metal/metal-oxide type, or of polarographic type, as is also preferable, all well known to the prior art. Once the electrodes 62-69 are formed, their interior analyte sensing ends remain exposed to an electrode channel 61 and to any sample contained therein. The electrode channel 61 is connected at one end to the electrode input tube 58 and at the other end to the waste tube 76 and is adapted to contain a sample being exposed to the electrodes 62-69. In one preferred embodiment the flow path of the electrode channels is rectilinear in cross-section (e.g., 1mm x 2mm) having a total volume of ca. 80 ul.

The electrode card 60 is backed by a metal plate 70 disposed adjacent to the electrode channel 61 which makes contact with a thermal unit 96 in the machine 80, allowing the microprocessor 100 to monitor and control the temperature of the sample while in the channel 61.

The exterior end of each of electrodes 62-69 is topped with an electrically conductive material. This conductive material is then drawn out to the distal end of the electrode card 60 to complete, upon insertion of the cartridge 20, the contact between the electrodes assaying the sample and the electrode interface 94 which connects to the microprocessor 100 contained in the machine 80. The microprocessor 100 is programmed to monitor, store, and display the assay results, among its other functions.

FIGURE 7 illustrates the peristaltic pump slot 74 which is disposed in the insertion end of the cartridge 20. The peristaltic pump slot 74 is a concave slot in the insertion end wall 50. The waste tube 76 running from the output end of the channel 61 to the bag fitting 26 of the waste collection bag 28 is brought out of the main body of the cartridge 20 through the insertion end wall 50 and suspended across the peristaltic pump slot 74. Upon insertion of the cartridge 20, the drive rollers 90 in the machine 80 pinch the exposed portion of the waste tube 76 against the concave wall of the slot 74. The rotation of the rollers 90 thus forces the test sample across the channel 61 of the electrode card 60, through the waste tube 76, and into the waste collection bag 28.

The Blood Chemistry Analysis Machine

In the preferred embodiment, the blood gas analysis machine 80 houses a selection valve driver means 82, a peristaltic pump driver means 88, a thermal unit 96, an electrode interface 94, a microprocessor 100, an operator keyboard 102, a printer 106, a display 104, an internal digital clock 108, and a back-up power source 110, as seen in the schematic diagram of FIGURE 1.

Power is provided to the blood gas analysis machine 80 by connection to a standard electrical outlet. A back-up power source 110, comprising a standard battery device which can power the system to maintain calibration for up to 30 minutes, is contained within the machine 80.

An internal digital clock 108 contained in the machine 80 is of standard design and provides a time base for the operation of the system.

The valve driver means 82, shown in FIGURES 1 and 8, which selectively directs either of the calibrating solutions or the test sample to the electrodes 62-69, preferably includes a rotatable shaft 84 which fits into the end of the bolt 46 of the selection valve 40. The position of the shaft 84 is controlled by the microprocessor 100 through a solenoid 86.

The peristaltic pump driver means 88, shown in FIGURES 1 and 8, which drives the fluid flow through the cartridge 20 comprises the rotatable peristaltic pump driver rollers 90 which contact a portion of the waste tube 76 suspended across the peristaltic pump slot 74 when the cartridge 20 is inserted into the blood gas analysis machine 80. The rotation of the driver rollers 90, powered by a motor 92, is controlled by the microprocessor 100.

The thermal unit 96 includes a resistance heater and a thermistor which are controlled by the microprocessor 100 to obtain a constant, predetermined temperature of samples in the electrode channel 61. Heat generated by the thermal unit 96 is conducted to the metal plate 70 adjacent to the channel 61.

The electrode interface 94, within the machine 80, connects to the electrodes 62-69 when the cartridge 20 has been inserted and selects one of the plurality of signals generated by the electrodes 62-69. This selected signal passes through to the microprocessor 100 which converts the signal from analog to digital form and then further processes the signal.

The microprocessor 100 is programmed to control those means described above and to control the printer 106 and the display 104; additionally, the microprocessor 100 receives, analyzes, and stores the calibrating and test sample signals from the electrodes 62-69.

The keyboard 102 is a standard keyboard device having a touch sensitive membrane which is mounted on the front panel and has a format as shown in FIGURE 9. The keyboard 102 allows the operator to initiate the input of the calibrating solution or the test sample to enter patient and operator identification information, to initiate print or display functions, to set the clock, to set the temperature, and to enter such data.

In the preferred embodiment, the display 104 is a standard, commercially available LED device, having a format shown in FIGURE 9. The display 104, controlled by the microprocessor 100, provides a constant reading of pH and of $CO_2$ and $O_2$ pressures in mmHg for the last sample from both the venous flow 14 and the arterial flow 15, as well as the operator's choice of hematocrit, $K^+$ or $Ca^{++}$ readings of the last sample. The display 104 can also provide readings of the current temperature, oxygen saturation, base excess, total $CO_2$, bicarbonate, oxygen consumption rate, or total blood volume consumed to date, as well as the status of the back-up power system, all available at the operator's discretion.

The printer 106 is a standard printer, such as a dot matrix or thermal printer, adapted to provide a hard copy of the time, date, patient and operator ID numbers, and temperature, as well as the values of all parameters of blood characteristics which can be the displayed by display 104, as described above.

Operation

In operation, power is provided to the blood chemistry analysis machine 80, and a cartridge 20 is inserted therein. The blood intake valve 30 is connected by the tubing 16 to the venous blood flow 14 and the arterial blood flow 15 of a conventional heart/lung machine 12 sustaining a surgical patient 10. An automatically operated valve 18 allows the operator to select between the venous flow 14 or the arterial flow 15. Inside the cartridge 20 and passing between the calibrating solution bags 22 and 24, a blood intake tube 32 connects a blood intake valve 30 to a selection valve 40.

Upon insertion of the cartridge 20 into the blood gas analysis machine 80, the bolt head 44 of the selection valve 40 connects to a shaft 84 in the machine 80, the electrode card 60 connects to the electrical contacts 95 in the machine 80 which lead to a microprocessor 100 contained therein, and the peristaltic pump slot 74 connects to the rotating drive rollers 90 in the machine 80. A metal plate 70 in the cartridge 20 connects to a thermal unit 96 of the machine 80, to monitor and control the temperature of samples in the electrode channel 61.

To initiate the automatic cycle of periodic analyses of the blood samples, the operator uses a keyboard 102 to enter the desired frequency of assays into the microprocessor 100. The microprocessor 100 then directs the shaft 84, which is in contact with the nut 44, to rotate a plug 42 of a selection valve 40, aligning an electrode input tube 58 with one of the calibrating solution ports 54 or 55. The port 54 is connected by tubing to one calibrating solution bag 22; the port 55 is connected to another calibrating solution bag 24. The microprocessor 100 selects first the calibrating solution in the first bag 22 and then the calibrating solution in the second bag 24 to establish a two-point calibration of the electrodes 62-69. Once the rotating plug 42 is appropriately positioned, the rotation of the rollers 90 along a portion of a waste tube 76, suspended across the peristaltic pump slot 74, draws the appropriate calibration solution into the channel 61 of the electrode card 60.

When either calibrating solution is in contact with the electrodes 62-69, a plurality of voltages or currents proportional to distinct ionic characteristics or gas concentrations of the solution pass from the electrodes 62-69 to an electrode interface 94 which selects one of the plurality of the signals. This selected signal passes to the microprocessor 100 which converts it from analog to digital form. In subsequent turns, the electrode interface 94 selects each of the other voltage signals. After the two-point calibration, the microprocessor 100 causes the rotating plug 42 to align the electrode input tube 58 with the blood sample port 52. The drive rollers 90 then draw a blood sample into the channel 61, at the same time forcing the calibration solution through the waste tube 76 and into the waste collection bag 28. The several voltage and current signals of the blood sample are measured, the distinct parameters are valued according to the two-point calibration and are displayed through appropriate means on the blood gas analysis machine 80. Additionally, the values of the distinct parameters of the blood sample may be stored in the microprocessor 100 for subsequent recall and display.

Constant temperature of samples in the channel 61 is insured by preprogramming the microprocessor 100 to monitor and control the temperature of a metal plate 70 through a thermal contact 97 and a thermal unit 96.

The calibration solution and blood sample assay sequence is repeated at intervals previously selected by the operator. For most subsequent interval assays, a one-point recalibration of the electrodes 62-69 is made; occasionally, a two-point recalibration is initiated by the microprocessor 100 to ensure continued accuracy.

Alternatively, a discrete blood sample may be connected to the blood intake port 30 and subjected to the above-outlined sequence, enabling the system to operate as a standard lab-based blood gas analyzer.

Following the exhaustion of calibrating solutions or following the termination of the surgical procedure of a particular patient, the spent cartridge 20 may be discarded and replaced with a new cartridge for subsequent use of the blood gas analysis system.

Therefore it is seen that this blood gas analysis system provides an economical, highly automated, contamination-free means to provide a surgeon with almost immediate information on the surgical patient's blood characteristics, which in turn reflects the patient's status.

**Claims**

1. A system for blood chemistry analysis comprising:

(a) a disposable cartridge (20) of the type that is replaceable after use, having a box-like body and having a fluid flow tube with a peristaltically pumpable tube section (76) external to the body, the body being adapted to be connected by insertion in operative engagement with a blood chemistry analysis machine (80) and to be disconnected after use and discarded, the body containing therein:

a first container (22) filled with a calibration blood facsimile liquid of known chemical characteristics including a known content therein of dissolved gas;

inlet means (30) mounted in the body to directly receive a blood sample;

a bank of ion sensing and gas sensing electrodes (62,69), each adapted to generate an electrical signal proportional to distinct chemical characteristics of said calibration liquid or blood test sample;

a fluid channel (61) that is open to said electrode bank and is adapted to be placed in open communication with the inlet means (30) or said liquid container (22);

selection valve means (40) operative to selectively direct the flow of either said calibration liquid or said blood sample over the bank of electrodes (62-69);

thermal plate (70) for maintaining the electrodes and the blood sample or calibration fluid in said channel at a constant temperature; and

(b) a blood chemistry analysis machine (80), adapted to receive said cartridge (20), said machine (80) including:

means (82) interconnecting with the cartridge to control said selection valve means (40) for selectively directing the flow of either said liquid of known chemical characteristics or said blood sample over the bank of electrodes (62-69) in the cartridge (20); and

means to receive (94) and analyze (100) the electrical signals generated by the bank of electrodes (62-69) upon exposure to said calibration liquid or said blood sample.

2. The system of claim 1 wherein said means (30) to receive a blood sample is adapted to be connected either to a container filled with a discrete sample or to a tube (16) in turn connected to the venous and arterial blood flows of a heart/lung machine (12) sustaining a patient 10) during surgery.

3. The system of claim 1 wherein said electrodes (62-69) comprise electrodes of a planar, solid-state structure.

4. The system of claim 1 wherein said first container (22) is a gas-impermeable bag.

5. The system of claim 1 wherein said cartridge includes a second container (24) filled with a calibration liquid of known chemical characteristics distinct from those of said calibration liquid contained in said first container (22).

6. The system of claim 5 wherein said second container (24) is a gas-impermeable bag.

7. The system of claim 1 wherein said cartridge (20) includes a waste container (28) adapted to receive liquid from the bank of electrodes (62-68) subsequent to analysis.

8. The system of claim 7 wherein said waste container (28) is a gas-permeable bag.

9. The system of claim 1 wherein said means (40) to selectively direct the flow of either said liquid of known chemical characteristic or said test sample over the bank of electrodes includes rotatable means (42) to block the flow of either said calibration liquid or said blood sample while allowing suitable flow of the selected fluid to the bank of electrodes (62-69), said selection being provided by means of a rotatable member (84), housed in said blood chemistry analysis machine and adapted to connect to said rotatable means.

10. The system of claim 1 wherein said means to receive and analyze said electrical signals includes a microprocessor (100).

11. The system of claim 1 wherein said means to receive and analyze said electrical signals includes means (94) to select any one of said plurality of electrical signals proportional to distinct chemical characteristics of a test sample.

12. The system of claim 1 wherein said machine includes a display means (104) connected to said means (100) to receive and analyze said electrical signals.

13. The system of claim 1 wherein said machine includes means (88) adapted to contact said cartridge (20) to provide force for the flow of liquid through said cartridge (20).

14. The system of claim 13 wherein said means to provide driving force for the flow of fluid comprises a peristaltic pump slot (74) in said cartridge (20), adapted to contact rotating drive rollers (90) of a peristaltic pump housed in said blood chemistry analysis machine (80).

15. The system of claim 1 wherein said machine (80) includes means (96) to detect and control the temperature of a sample in contact with said bank of electrodes.

16. The system of claim 15 wherein said temperature control means comprises a metal plate (70), adjacent to said bank of electrodes (62-69), adapted to connect, through thermally conductive contacts, to a thermal unit (96) housed in said blood chemistry analysis machine (80).

17. The system of claim 1, including a cartridge having a plurality of sensors (62-69) disposed upon an electrode card (60) which is backed by a metal plate (70).

18. The system of claim 17 wherein said sensors comprise ion sensing and gas sensing electrodes (62-69).

19. The system according to claim 1, wherein said cartridge supports two containers (22,24) each including a calibration solution such that the cartridge is operative for two-point calibration.

**Revendications**

1. Système pour l'analyse de la chimie du sang comprenant:
   [a] une cartouche à jeter [20] du type qui peut être remplacé après usage, ayant un corps en forme de boîte et comprenant un tube pour l'écoulement de liquide avec une section de tube pompable péristaltiquement [76] externe au corps, le corps étant adapté pour être raccordé par insertion avec mise en prise de façon à fonctionner avec une machine pour l'analyse de la chimie du sang [80] et pour être détaché après emploi et rejeté le corps contenant:
   un premier récipient [22] rempli d'un liquide facsimilé de sang d'étalonnage ayant des caractéristiques chimiques connues dans lequel est dissous une quantité connue de gaz;
   un moyen d'entrée [30] monté dans le corps pour recevoir directement un échantillon de sang;
   une batterie d'électrodes pour détecter les ions et pour détecter les gaz [62,69] chacune étant adaptée pour générer un signal électrique proportionnel à des caractéristique chimiques distinctes de ce liquide d'étalonnage ou de cet échantillon d'essai de sang;
   un canal de liquide [61] qui ouvre sur cette batterie d'électrodes et est adapté pour être placé en communication ouverte avec le moyen d'entrée [30] ou ce récipient de liquide [22];
   un moyen de vanne de sélection [40] fonctionnant pour diriger sélectivement l'écoulement de ce liquide d'étalonnage ou de cet échantillon de sang sur la batterie d'électrodes [62-69];
   une plaque thermique [70] pour maintenir les électrodes et l'échantillon de sang ou le liquide d'étalonnage dans ce canal à une température constante; et
   [b] une machine [80] pour l'analyse de la chimie du sang, adaptée pour recevoir cette cartouche [20], cette machine comprenant:
   un moyen [82] d'interconnexion avec la cartouche pour contrôler ce moyen de vanne de sélection [40] de façon à diriger sélectivement l'écoulement de ce liquide ayant des caractéristiques chimiques connues ou de cet échantillon de sang sur la batterie d'électrodes [62-69] dans la cartouche [20], et
   un moyen pour recevoir [94] et analyser [100] les signaux électriques générés par la batterie d'électrodes [62-69] lors de l'exposition à ce liquide d'étalonnage ou à cet échantillon de sang.

2. Système suivant la revendication 1, dans lequel ce moyen [30] pour recevoir un échantillon de sang est adapté pour être raccordé soit à un récipient rempli d'un échantillon discret soit à un tube [16] lequel est raccordé aux flux veineux et artériel d'une machine coeur/poumon [12] entretenant un patient [10] pendant un acte chirurgical.

**3.** Système suivant la revendication 1, dans lequel ces électrodes [62-69] comprennent des électrodes de structure planaire, à l'état solide.

**4.** Système suivant la revendication 1, dans lequel ce premier récipient [22] est un sac imperméable aux gaz.

**5.** Système suivant la revendication 1, dans lequel cette cartouche comprend un second récipient [24] rempli d'un liquide d'étalonnage de caractéristiques chimiques connues distinctes de celles de ce liquide d'étalonnage contenu dans ce premier récipient [22].

**6.** Système suivant la revendication 5, dans lequel ce second récipient [24] est un sac imperméable aux gaz.

**7.** Système suivant la revendication 1, dans lequel cette cartouche [20] comprend un récipient à déchets (28) adapté pour recevoir le liquide provenant de la batterie d'électrodes (62-68] après analyse.

**8.** Système suivant la revendication 7, dans lequel ce récipient à déchets [28] est un sac imperméable aux gaz.

**9.** Système suivant la revendication 1, dans lequel ce moyen [40] pour diriger sélectivement l'écoulement de ce liquide de caractéristique chimique connue ou de cet échantillon d'essai sur la batterie d'électrodes, comprend un moyen pouvant tourner [42] pour bloquer l'écoulement de ce liquide d'étalonnage ou de cet échantillon de sang tout en permettant un écoulement convenable du liquide sélectionné vers la batterie d'électrodes [62-69], cette sélection étant fournie à l'aide d'un élément pouvant tourner [84] logé dans cette machine pour l'analyse de la chimie du sang et adapté pour être raccordé à ce moyen pouvant tourner.

**10.** Système suivant la revendication 1, dans lequel ces moyens pour recevoir et analyser ces signaux électriques comprennent un microprocesseur [100].

**11.** Système suivant la revendication 1, dans lequel ces moyens pour recevoir et analyser ces signaux électriques comprennent un moyen [94] pour sélectionner l'un quelconque de ces nombreux signaux électriques proportionnels à des caractéristiques chimiques distinctes d'un échantillon d'essai.

**12.** Système suivant la revendication 1, dans lequel cette machine comprend un moyen d'affichage [104] raccordé à ce moyen [100] pour recevoir et analyser ces signaux électriques.

**13.** Système suivant la revendication 1, dans lequel cette machine comprend un moyen [88] adapté pour entrer en contact avec cette cartouche [20] afin de fournir une force pour l'écoulement du liquide à travers cette cartouche (20).

**14.** Système suivant la revendication 13, dans lequel ce moyen pour fournir une force d'entraînement pour l'écoulement de fluide comprend une fente de pompe péristaltique [74] dans cette cartouche [20], adaptée pour entrer en contact avec des cylindres d'entraînement rotatifs [90] d'une pompe péristaltique logée dans cette machine [80] pour l'analyse de la chimie du sang.

**15.** Système suivant la revendication 1, dans lequel cette machine [80] comprend un moyen [96] pour détecter et contrôler la température d'un échantillon en contact avec cette batterie d'électrodes.

**16.** Système suivant la revendication 15, dans lequel ce moyen de contrôle de la température comprend une plaque métallique [70], adjacente à cette batterie d'électrodes (62-69) adaptée pour être raccordée, par des contacts conduisant la chaleur, à une unité thermique [96] logée dans cette machine [80] pour l'analyse de la chimie du sang.

**17.** Système suivant la revendication 1, comprenant une cartouche ayant un certain nombre de capteurs [62-69] disposés sur une carte d'électrodes [60] qui est soutenue par une plaque métallique [70].

**18.** Système suivant la revendication 17, dans lequel ces capteurs comprennent des électrodes détectant des ions et détectant des gaz [62-69].

**19.** Système suivant la revendication 1, dans lequel cette cartouche supporte deux récipients [22,24] comprenant chacun une solution d'étalonnage de telle sorte que la cartouche fonctionne pour un étalonnage à deux points.

**Patentansprüche**

**1.** System für die Blutchemieanalyse, aufweisend,

a) eine wegwerfbare Kartusche (20) des Typs, der nach der Verwendung ersetzbar ist, mit einem kastenförmigen Körper und einer Fluiddurchflußleitung mit einem peristaltisch pumpbaren Schlauchabschnitt (76), der außerhalb des Körpers angeordnet ist, wobei der Körper durch Einlegen in Arbeitseingriff mit einer Blutchemieanalysemaschine (80) verbunden und nach Verwendung abgetrennt und verworfen werden kann und der Körper hierin enthält

einen ersten Behälter (22), der mit einer dem Blut nachgebildeten Kalibrierungsflüssigkeit bekannter chemischer Charakteristika einschließlich eines bekannten Gehalts von hierin gelöstem Gas gefüllt ist,

Einlaßmittel, die in dem Körper befestigt sind, um direkt eine Blutprobe aufzunehmen;

eine Reihe von ionen- und gassensitiven Elektroden (62, 69), die jeweils geeignet sind, ein elektrisches Signal zu erzeugen, das proportional zu bestimmten chemischen Charakteristika der Kalibrierungsflüssigkeit oder der Bluttestprobe ist,

einen Flüssigkeitskanal (61), der zur Elektrodenreihe hin offen ist und geeignet ist, in offener Strömungsverbindung mit dem Einlaßmittel (30) oder dem Flüssigkeitsbehälter (22) angeordnet zu werden;

eine einstellbare Ventilanordnung (40), die dahingehend betätigbar ist, daß selektiv der Fluß entweder der Kalibrierungsflüssigkeit oder der Blutprobe über die Elektrodenreihe (62-69) gerichtet wird;

eine temperierte Platte (70), um die Elektroden und die Blutprobe oder die Kalibrierungsflüssigkeit im Kanal bei einer konstanten Temperatur zu halten; und
b) eine Blutchemieanalysemaschine (80), die geeignet ist, die Kartusche (20) aufzunehmen, wobei die Maschine (80) umfaßt: Mittel (82) zur Verbindung mit der Kartusche, um die wählbare Ventilanordnung (40) so zu steuern, daß selektiv der Fluß entweder der Flüssigkeit mit bekannten chemischen Charakteristika oder der Blutprobe über die Elektrodenreihe (62-69) in der Kartusche (20) gerichtet wird, und

Mittel zum Empfang (94) und Analysieren (100) der elektrischen Signale, die durch die Elektrodenreihe (62-69) bei Beaufschlagung durch die Kalibrierungsflüssigkeit oder die Blutprobe erzeugt werden.

**2.** System nach Anspruch 1, bei dem der Einlaß (30) zur Entgegennahme einer Blutprobe geeignet ist, daß er entweder an einen mit einer bestimmten Probe gefüllten Behälter oder an einen Schlauch (16) angeschlossen wird, der wiederum an die venösen und arteriellen Leitungen einer Herz/Lungen-Maschine (12) angeschlossen ist, die einen Patienten (10) während einer Operation am Leben hält.

**3.** System nach Anspruch 1, bei dem die Elektroden (62-69) Elektroden einer ebenen Festkörperstruktur umfassen.

**4.** System nach Anspruch 1, bei dem der erste Behälter (22) ein gasundurchlässiger Beutel ist.

**5.** System nach Anspruch 1, bei dem die Kartusche einen zweiten Behälter (24) umfaßt, der mit einer Kalibrierungsflüssigkeit bekannter chemischer Charakteristika gefüllt ist, die von denjenigen der Kalibrierungsflüssigkeit verschieden ist, die in dem ersten Behälter (22) enthalten ist.

**6.** System nach Anspruch 5, bei dem der zweite Behälter (24) ein gasundurchlässiger Beutel ist.

**7.** System nach Anspruch 1, bei dem die Kartusche (20) einen Abfallbehälter (28) umfaßt, der geeignet ist, Flüssigkeit von der Elektrodenreihe (62-68) im Anschluß an die Analyse aufzunehmen.

**8.** System nach Anspruch 7, bei dem der Abfallbehälter (28) ein gasdurchlässiger Beutel ist.

**9.** System nach Anspruch 1, bei dem die Mittel (40) zur selektiven Ausrichtung des Flusses entweder der Flüssigkeit bestimmter chemischer Zusammensetzungen oder der Testprobe über die Elektrodenreihe ein drehbares Mittel (42) umfaßt, um den Fluß entweder der Kalibrierungsflüssigkeit oder der Blutprobe zu blockieren, während ein geeigneter Fluß des gewählten Fluids zu der Elektrodenreihe (62-69) gestattet ist, wobei die Einstellung mittels eines drehbaren Glieds (84) vorgenommen wird, das in der Blutchemieanalysemaschine angeordnet ist und geeignet ist, mit dem drehbaren Mittel verbunden zu werden.

**10.** System nach Anspruch 1, bei dem die Mittel zum Empfang und Analysieren der elektrischen Signale einen Mikroprozessor (100) umfassen.

**11.** System nach Anspruch 1, bei dem die Mittel zum Empfang und Analysieren der elektrischen Signale Mittel (94) zur Selektion jedes der Vielzahl von elektrischen Signalen umfassen, die proportional zu bestimmten chemischen Charakteristika einer Testprobe sind.

**12.** System nach Anspruch 1, bei dem die Maschine ein Anzeigemittel (104) umfaßt, das mit dem Mittel (100) zum Empfang und Analysieren der elektrischen Signale verbunden ist.

**13.** System nach Anspruch 1, bei dem die Maschine Mittel (88) aufweist, die zur Herstellung eines Kontakts mit der Kartusche (20) geeignet sind, um Energie für den Fluß der Flüssigkeit durch die Kartusche (20) zur Verfügung zu stellen.

**14.** System nach Anspruch 13, bei dem das Mittel zur Bereitstellung der Antriebsenergie für den Fluß der Flüssigkeit eine peristaltische Pumpenausnehmung (74) in der Kartusche (20) aufweist, die mit den sich drehenden Antriebssrollen (90) der peristaltischen Pumpe, die in der Blutchemieanalysemaschine (80) angeordnet ist, in Kontakt treten kann.

**15.** System nach Anspruch 1, bei dem die Maschine (80) Mittel (96) zur Feststellung und Steuerung der Temperatur einer Probe bei Kontakt mit der Elektrodenreihe umfaßt.

**16.** System nach Anspruch 15, bei dem das Temperatursteuerungsmittel eine Metallplatte (70) benachbart zu der Elektrodenreihe (62-69) umfaßt, die durch thermisch leitende Kontakte an eine thermische Einheit (96) anschließbar ist, die in der Blutchemieanalysemaschine untergebracht ist.

**17.** System nach Anspruch 1, umfassend eine Kartusche mit einer Vielzahl von Sensoren (62-69), die auf einer Elektrodenkarte (60) angeordnet sind, die auf ihrer Rückseite von der Metallplatte (70) abgedeckt ist.

**18.** System nach Anspruch 17, bei dem die Sensoren ionensensitive und gassensitive Elektroden (62-69) umfassen.

**19.** System nach Anspruch 1, bei dem die Kartusche zwei Behälter (22, 24) trägt, die jeweils eine Kalibrierungslösung derart aufweisen, daß die Kartusche für eine Zweipunktkalibrierung ausgelegt werden kann.

Fig-1

EP 0 226 593 B1

*Fig-2*

*Fig-7*

*Fig-4*

*Fig-5*

*Fig-3*

*Fig-8*

THERMAL UNIT

M/P

MOTOR

SOLENOID

*Fig-6A*

*Fig-6B*

*Fig-9*